# EUROPEAN PATENT SPECIFICATION

(11) **EP 1 598 366 B1**
(45) Date of publication and mention of the grant of the patent: **20.08.2008**
(21) Application number: 05101866.1
(22) Date of filing: 10.03.2005
(51) Int. Cl.: C07K 14/655, A61K 38/31, A61P 35/00

(54) **Somatostatin analogues**
Analoge des Somatostatin
Analogues de la somatostatine

(30) Priority: 10.03.2004 IT FI20040057
(43) Date of publication of application: 23.11.2005
(73) Proprietor: Advanced Accelerator Applications, 01630 Saint Genis Pouilly (FR)
(72) Inventor: Giuntini, Mauro, 50139 Firenze (IT); Ginanneschi, Mauro, 59100 Prato (IT); Chelli, Mario, 50064 Incisa Val d'Arno (IT); Papini, Anna Maria, 50127 Firenze (IT)
(74) Representative: Gervasi, Gemma

(56) References cited:
- US-A1- 2002 052 315
- WHELAN A N ET AL: "A tandem metathesis-hydrogenation route to dicarba analogues of cystine-containing cyclic peptides" TETRAHEDRON LETTERS, ELSEVIER SCIENCE PUBLISHERS, AMSTERDAM, NL, vol. 45, no. 52, 20 December 2004 (2004-12-20), pages 9545-9547, XP004718540 ISSN: 0040-4039

### SEQUENCE LISTING

<110> Giuntini, Mauro et al
<120> Somatostatin analogues
<130> 4902PTEP
<140> EP 05101866.1
   <141> 2005-03-10
<150> FI2004A000057
   <151> 2004-03-10
<160> 1
<170> PatentIn version 3.3
<210> 1
   <211> 8
   <212> PRT
   <213> Artificial
<220>
   <223> Synthetic octopeptide - Formula I
<220>
   <221> MISC_FEATURE
   <222> (2)..(2)
   <223> Allylic Glycine, used in peptide cyclisation reaction
<220>
   <221> MISC_FEATURE
   <222> (7)..(7)
   <223> Allylic Glycine, used in peptide cyclisation reaction
<400> 1
   Phe Gly Phe Trp Lys Thr Gly Thr
   1               5

## Description

### Technical field

The invention described herein relates to compounds analogue of Somatostatin.

### State of the art

It is well known that Somatostatin is an hormone and its biological regulatory activity plays an important role as this substance inhibits the release of several others hormones as glucagon, growth hormone, insulin, gastrin. In addition, because of its distribution in the central nervous system and in the spinal cord this peptide can play a role in the neuronal transmission.

However, the use of Somatostatin as a drug is hampered by its very short half-life in vivo (2 or 3 min). This fact prompted an intense and continue research of agonists of this hormone for pharmacological applications in different fields of Medicine, from cancer and acromegaly therapy to therapy of diabetes, rheumatoid arthritis and of Alzheimer disease.

Up to now, many Somatostatin analogues have been discovered, some very potent, generally formed from cyclic peptides of six to eight amino acids.

Among them, should be mentioned Sandostatin or octreotide which showed long half-life time in vivo (from 60 to 90 min) and it is 2000 times more active than Somatostatin itself. This product is also a carrier of radioisotopes when conjugated with chelating agents as DOTA (DOTA-TOC) for diagnosis and radiotherapy of tumours.

However, despite the above mentioned advantages, also octreotide did not always showed the desired effects mainly as far as it concerns the anti mitotic efficiency towards the breast, prostate and colon tumours. More, the disulphide bridge of this molecule is subjected to the attack of reducing endogenous agents (i. e. glutathione oxidase or thioredoxin redictase) and exogenous oxidants as well as of basic and nucleophilic agents.

US2002/0052315 discloses derivatives of octreotide, wherein the disulphide bridge has been replaced by i.a. an amide, thioether, thioester group.

As a consequence of the arguments above reported it appears that it is very important the preparation of new agonists of Somatostatin for therapeutic purpose.

### Detailed description of the invention

The present invention allows the resolution of the above mentioned problem thank to formula (I) compounds as below defined: 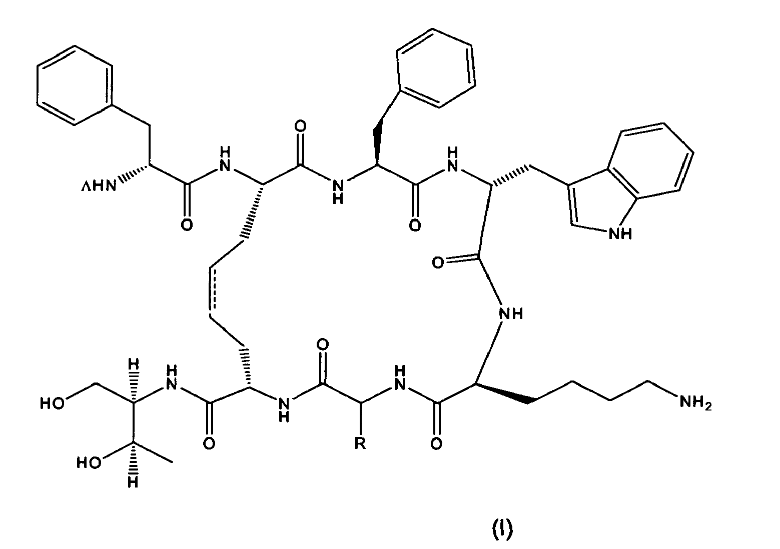 where the symbol ----- is a simple or a double bond;
-A is H, the chelating agent DTPA or a macrocycle of formula (II): 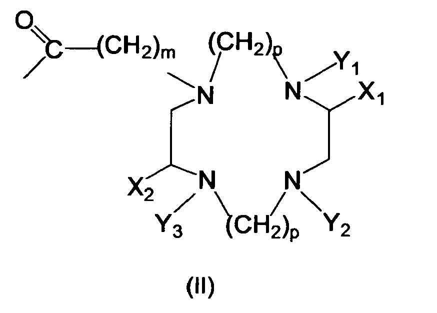 where Y₁, Y₂, and Y₃, which may be the same or different, are chosen in the group consisting of hydrogen, -(CH₂)ₙ-COOH, where n is an integer from 1 to 6, and p is the integer 2 or 3.
R is an amino acid as Thr, D- or L-Phe otherwise D- or L-Phe orto and/or para substituted with -OR' groups where R' is straight or branched alkyl, benzyl or benzyl substituted on the aromatic ring with hydrophobic groups.

An example of macrocycle of formula (II) according to the invention is the chelating agent DOTA.

The compound object of the invention described herein is prepared by using known methods and reagents which are known or which can be obtained by methods known to those skilled in the art.

In particular, as far the preparation process is concerned:
(a) -in the first step the linear peptide is prepared by solid phase peptide synthesis as it is well known in the art (the amino acids, the resin and the needed reagents are commercially available or can be synthesised by those skilled in the art);
(b) -after de-protection, the on-resin linear peptide is cyclised through a first generation Grubbs catalyst;
(c)-the cyclopeptide is cleaved from the resin and purified by RP-HPLC;
possibly
(d) -after the cleavage, the cyclopeptide is reduced in solution by H₂ in the presence of a metal catalyst and is purified by RP-HPLC;
otherwise after step (b)
(c')- the cyclopeptide is reduced on-resin by H₂ in the presence of a metal catalys
(d') the reduced cyclopeptide is cleaved from the resin and is purified by RP-HPLC. otherwise -the unsaturated cyclopeptide might be conjugated to the formula (II) macrocycle, at the level of the terminal amino group of the D-Phe¹, by treating the on-resin cyclopeptide with 20% piperidine in DMF and by adding a solution of the macrocycle suitably activated following the methods known in the literature (i. e. G. Sabatino, M. Chinol, G. Paganelli, S. Papi, M. Chelli, G. Leone, A. M. Papini, A. De Luca, M. Ginanneschi, J. Med. Chem., 2003, 46, 3170-3173; M. Scottelius, M. Schwaiger, HJ. Wester, Tetrahedron Lett., 2003, 44, 2393-2396; J. Ficnha, A. Janecka, Bioconjugate. Chem., 2003, 14, 3-17);
   possibly
   also the saturated cyclic peptide, if obtained by reducing the on-resin unsaturated peptide, might be conjugated with a formula (II) macrocycle under the same conditions.

For the synthesis of the linear peptide we followed in general the method reported by Wu et al. (Y-T. Wu, H-P. Hsieh, C-Y.Wu, S-T. Tein, K-T. Wang, Tetrahedron Letters, 1998, 39, 1783-1984).

In the first step the linear peptide Fmoc-D-Phe-Gly(All)-Phe-D-Trp(Boc)-Thr(tBu)-Gly(All)-Thr-ol is synthesised by solid phase peptide synthesis in batch, by using a Rink Amide resin [(4-(2',4'-dimethoxyphenyl-Fmoc-aminomethyl))-phenoxy resin, 0.44 mmol/g from Novabiochem].

Preferably four equivalents of the necessary amino acids, protected with Fmoc and activated by HOBt/TBTU/NMM in DMF, are used.

The first amino acid bonded to the resin (the linker) is the acetal obtained by condensation of Fmoc-threoninol and p-carboxyaldehyde.

Once the linker has been synthesised, the next steps are like it has been above reported. The following examples further illustrate the synthetic pathway of the invention.

### Example n. 1.

### Synthesis of the linker (Fmoc-threoninol-p-carboxybenzaldehyde acetal)

Solution A: 0.9 mmol of Fmoc-threoninol (300 mg) in 10 mL of CHCl₃ (dried on molecular sieves).

Solution B: 2.25 mmol of p-carboxybenzaldehyde (340 mg) in 10 mL of CHCl₃.

To the solution B some drops of DMSO and a very small amount of p-toluensulphonic acid are added.

The solution A is added to the solution B under stirring; afterwards, 10 mL of CHCl₃ are also added and then the solution is heated up to 80 °C and refluxed for 8 h under nitrogen in a Dean-Stark apparatus.

The solution is then cooled to room temperature and evaporated under reduced pressure obtaining an yellow oil.

The product is purified by flash chromatography on silica gel; eluent: CHCl₃/MeOH = 13:1.

The pure compound has been collected in 65% yield.

¹H- NMR (CDCl₃), δ 8.13 (2H, d, 8.3Hz), 7.75 (2H, d, 7.5Hz), 7.60-7.55 (4H , m), 7.41-7.28 (4H, m) 5.63-5.56 (2H, m), 4.47-4.42 (2H, m), 4.25-4.12 (4H, m), 3.71 (1H, d), 1.27 (3H, m, -CH₃).

ESI-MS [M+H]⁺ 460.4 calculated 460.17.

### Example n. 2

### Synthesis of the linear peptide

D-Phe -Gly(All)-Thr-Lys-D-Trp-Phe-Gly(All)-Thr-ol

The synthesis of the linear peptide was carried out by using 1 g of the resin previously deprotected with 20% piperidine in DMF. The necessary amount of the linker is dissolved in DMF, added of the activating agents and joined to the resin that is kept oscillating for 2 h. Next the resin is washed with DCM and DMF. The Kaiser test has put in evidence the presence of free amino groups which have been acetylated by a mixture of acetic anhydride and NMM. Then, all the remaining amino acids have been added to the resin suspended in DMF as previously described for the linker and following the planned sequence, carrying out a de-protection cycle with piperidine for each added amino acid except for the last. The final level of substitution of the resin is 0.2 mmol/g.

The linear peptide is analysed after a micro-cleavage on 5 mg of the resin.

The cleavage is at the same time a de-protection of the side-chains of Lys, Trp and Thr but the Fmoc terminal protecting group is not removed.

The resin is treated with 1 mL of a solution of 9 mL of TFA, 4 mL of water, 0.5 mL of phenol and 1 mL of ethaneditiol.

The resin is filtered off and the solution is evaporated under N₂ stream and the peptide is precipitated by treating with cold ethyl ether. The product is collected by centrifugation and then is dissolved in H₂O and lyophilised.

The product is analysed by RP-HPLC on a analytical column Phenomenex C₁₈ (250 mm x 4.6 mm internal diameter) by using a gradient 20% to 100% of eluent B (A: 0.1 %TFA in H₂O; B: CH₃CN 84%, 0.1 % TFA, 1 mL/min) Rₜ = 9.6 min.

ESI-MS: [M+H]⁺ 1231.9; calculated 1230.6.

### Example n. 3

### Synthesis of the unsaturated cyclic peptide

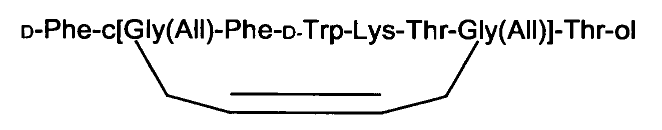

The cyclisation has been carried out on the peptide bonded to the resin through metathesis of two allyl residues of the glycine following the method reported by Miller et al. (S. J. Miller, H. E. Blackwell, R. H. Grubbs, J. Am. Chem. Soc., 1996, 118, 9606-9614).

To the resin (1 g) suspended in DCM (90 mL) the Grubbs catalyst [Ru(Cl)₂(Pcy₃)benzylidene, 43 mg] in DCM (13 mL) is added under nitrogen fluxing. The suspension is left at 40 °C for 30 h, then the resin is washed, dried and the cyclopeptide is Fmoc deprotected. Then the cyclopeptide is cleaved from the resin following the above mentioned procedure. The crude product is purified by preparative RP-HPLC [Vydac C18 ODSSTP1010 column, 250x25 mm i.d.; eluent: (A) 0.1% TFA in H₂O and (B) 0.1% TFA in CH₃CN, 15-60% of B in 30 min]. From 500 mg of resin are obtained 23 mg (25% yield) of a colourless solid that shows only one chromatographic peak.

ESI-MS: [M+H]⁺ 981.8; calculated 981.5.

### Example n. 4

### Synthesis of the saturated cyclic peptide

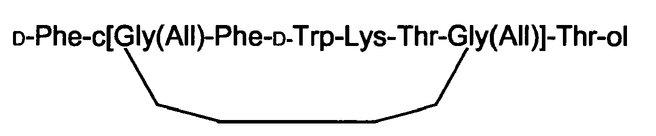

The unsaturated cyclic peptide (19 mg) is dissolved in MeOH and reduced under hydrogen atmosphere by using Pd(OH)₂/C as a catalyst, at room pressure and temperature, during 24 h.

The crude product is purified by preparative RP-HPLC using the same conditions as for the unsaturated product, obtaining 5 mg (26 % yield) of the desired compound.

ESI-MS: [M+H]⁺ 983.6; calculated 983.5.

The products above described, if -A is H, can be obtained for the preparation of pharmaceutical compositions following the usual methods known in the pharmaceutical technology for the use of equivalent active components.

Otherwise, the formula (I) products, if -A is a macrocycle of formula (II), can be radiolabelled with metal isotopes as Fe-52, Mn-52m, Co-55, Cu-64, Ga-67, Ga-68, Tc-99m, In-111, I-123, I-125, I-131, P-32, Sc-47, Cu-67, Y-90, Pd-109, Ag-111, Pm-149, Re-186, Re-188, At-211, Bi-212, Bi-213, Rh-105, Sm-153, Lu-177, and Au-198, used in radio-diagnosis and radio-therapy.

The above mentioned pharmaceutical compositions could be useful for the treatment of diseases as, for example, tumours, acromegaly, diabetes, rheumatoid arthritis, and Alzheimer disease.

## Claims

1. Compounds of formula (I) 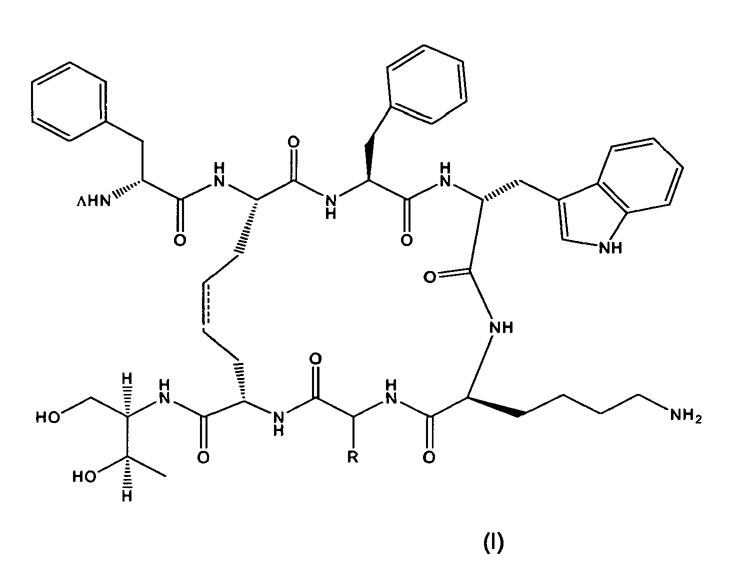 where the symbol ----- is a simple or a double bond; -A is H, the chelating agent DTPA or a macrocycle of formula (II): 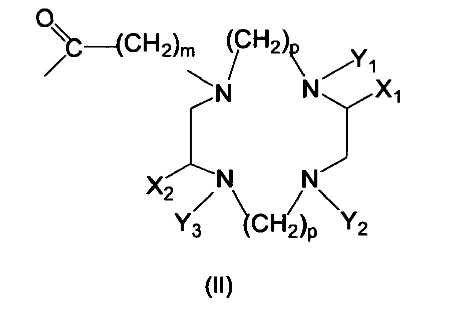 where Y₁, Y₂, and Y₃, which may be the same or different, are chosen in the group consisting of hydrogen, -(CH₂)ₙ-COOH, where n is an integer from 1 to 6, and p is the integer 2 or 3.
R is an amino acid as Thr, D- or L-Phe otherwise D- or L-Phe orto and/or para substituted with -OR' groups where R' is straight or branched alkyl, benzyl or benzyl substituted on the aromatic ring with hydrophobic groups.

2. Compound of formula (I) according to Claim 1 wherein the symbol ----- is a simple bond

3. Compound of formula (I) according to Claim 1 wherein the symbol ----- is a double bond

4. Compound according to claims 1- 3 wherein the macrocycle of formula (II) is the chelating agent DOTA.

5. Process for the preparation of compounds according to claims 1-4 wherein -A is H, wherein:
(a) -in the first step the linear peptide is prepared by solid phase peptide synthesis as it is well known in the art;
(b) -after de-protection, the on-resin linear peptide is cyclised through a first generation Grubbs catalyst;
(c)-the cyclopeptide is cleaved from the resin and purified by RP-HPLC;
possibly
(d) -after the cleavage, the cyclopeptide is reduced in solution by H₂ in the presence of a metal catalyst and is purified by RP-HPLC;
or after step (b)
(e)- the cyclopeptide is reduced on-resin by H₂ in the presence of a metal catalys
(f) the reduced cyclopeptide is cleaved from the resin and is purified by RP-HPLC.

6. Process for the preparation of compounds according to claims 1-4 wherein -A is a macrocycle of formula (II), wherein:
(a) -in the first step the linear peptide is prepared by solid phase peptide synthesis;
(b) -after de-protection, the on-resin linear peptide is cyclised through a first generation Grubbs catalyst;
(c) - the unsatured cyclopeptide is treated with piperidine;
(d) the cyclopeptide is conjugated with a solution of the activated macrocycle of formula (II);
(e) -the bioconjugated compound is cleaved from the resin and purified by RP-HPLC; or, after step (b):
(c')- the cyclopeptide is reduced on-resin;
(d') - the satured cyclopeptide is treated with piperidine;
(e') -the cyclopeptide is conjugated with the macrocyclo;
(f) the bioconjugated compound is cleaved from the resin and is purified by RP-HPLC.

7. Pharmaceutical formulations containing as active principle a compound of formula (I).

8. Use of a compound of formula (I) for the preparation of pharmaceutical formulations useful in the treatment of: tumours, acromegaly, diabetes, rheumatoid arthritis, and Alzheimer disease.

## Patentansprüche

1. Verbindungen der Formel (I) 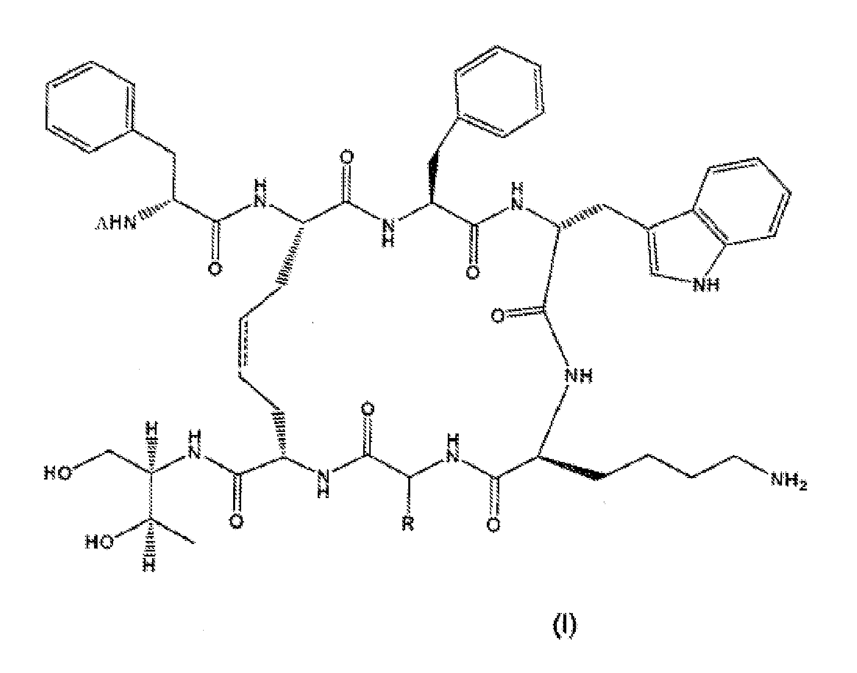 wobei das Symbol ----- für eine Einfach- oder Doppelbindung steht;
^{-Λ} ist H, der Chelatbildner DTPA oder ein Makrozyklus von Formel (II): 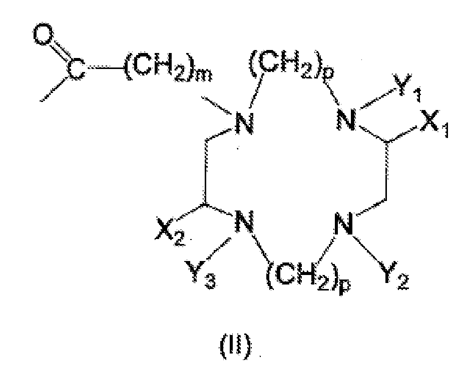 wobei Y₁, Y₂ und Y₃, welche gleich oder unterschiedlich sein können, aus der Gruppe ausgewählt werden, die aus Wasserstoff, -(CH₂)ₙ-COOH besteht, wobei n eine ganze Zahl von 1 bis 6 und p die ganze Zahl von 2 oder 3 ist.
R ist eine Aminosäure wie beispielsweise Thr, D- oder L-Phe ansonsten D- oder L-Phe ortho-und/oder para-substituiert durch -OR'-Cruppen, wobei R' für geradkettiges oder verzweigtes Alkyl, Benzyl oder Benzyl, welches am aromatischen Ring durch hydrophobe Gruppen substituiert ist, steht.

2. Verbindung der Formel (I) gemäß Anspruch 1, wobei das Sylnbol ----- für eine Einfachbindung steht.

3. Verbindung der Formel (I) gemäß Anspruch 1, wobei das Symbol ----- für eine Doppelbindung steht.

4. Verbindung gemäß Ansprüchen 1 - 3, wobei es sich bei dem Makrozyklus von Formel (II) um den Chelatbildner DOTA handelt.

5. Verfahren für die Herstellung von Verbindungen gemäß Ansprüchen 1 - 4, wobei ^{-Λ} H ist, wobei:
(a) in dem ersten Schritt das lineare Peptid mithilfe von Festphasen-Peptidsynthese, wie sie auf dem Fachgebiet gut bekannt ist, hergestellt ist;
(b) nach dem Entschützen das auf dem Harz befindliche lineare Peptid durch eine erste Generation von Grubbs-Katalysatoren zyklisiert ist;
(c) das Cyclopeptid von dem Harz abgespalten und mittels RP-HPLC gereinigt ist; möglicherweise
(d) nach der Abspaltung das Cyclopeptid in Lösung durch H₂ in Anwesenheit von einem Metallkatalysator reduziert ist und mittels RP-HPLC gereinigt ist:
oder nach Schritt (b)
(e) das Cyclopeptid auf dem Harz durch H₂ in Anwesenheit von einem Metallkatalysator reduziert ist;
(f) das reduzierte Cyclopeptid von dem Harz abgespalten ist und mittels RP-HPLC gereinigt ist.

6. Verfahren für die Herstellung von Verbindungen gemäß Ansprüchen 1 - 4, wobei ^{-Λ} ein Makrozyklus von Formel (II) ist, wobei:
(a) in dem ersten Schritt das lineare Peptid mithilfe von Festphasen-Peptidsynthese hergestellt ist;
(b) nach dem Entschützen das auf dem Harz befindliche lineare Peptid durch eine erste Generation von Grubbs-Katalysatoren zyklisiert ist;
(c) das ungesättigte Cyclopeptid mit Piperidin behandelt ist;
(d) das Cyclopeptid mit einer Lösung des aktivierten Makrozyklus von Formel (II) konjugiert ist;
(e) die biokonjugierte Verbindung von dem Harz abgespalten ist und mittels RP-HPLC gereinigt ist;
oder nach Schritt (b):
(c') - das Cyclopeptid auf dem Harz reduziert ist;
(d') - das gesättigte Cyclopeptid mit Piperidin behandelt ist;
(e') - das Cyclopeptid mit dem Makrozyklus konjugiert ist;
(f) die biokonjugierte Verbindung von dem Harz abgespalten ist und mittels RP-HPLC gereinigt ist.

7. Pharmazeutische Formulierungen, welche als Wirkstoff eine Verbindung von Formel (I) enthalten.

8. Verwenden einer Verbindung von Formel (I) für die Herstellung von pharmazeutischen Formulierungen, welche bei der Behandlung von Tumoren, Akromegalie, Diabetes, rheumatoider Arthritis und Alzheimer-Krankheit hilfreich sind.

## Revendications

1. Composés de formule (I) 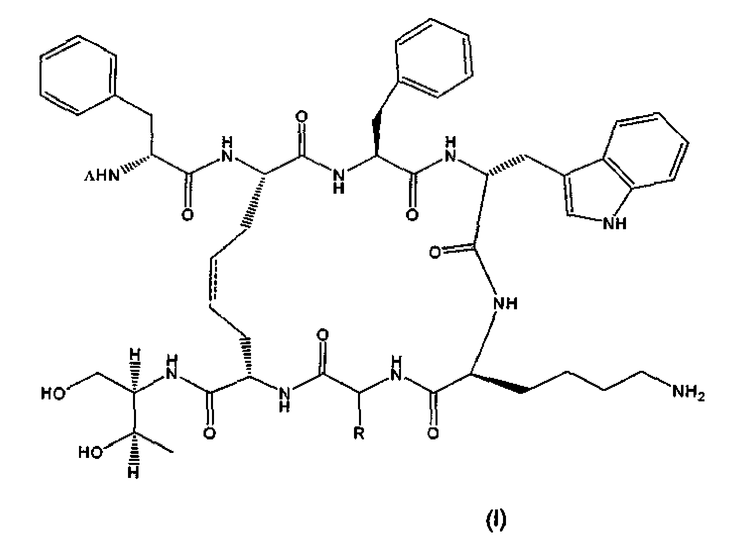 dans laquelle le symbole ------ est une liaison simple ou une double liaison ;
-A est H, l'agent chélatant DTPA ou un macrocycle de formule (II) : 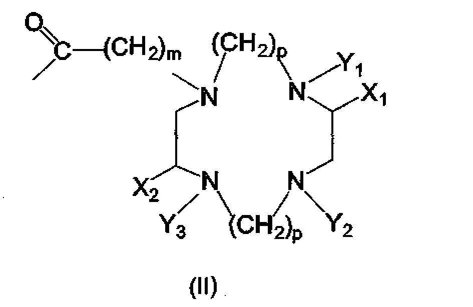 dans laquelle
Y₁, Y₂ et Y₃, qui peuvent être identiques ou différents sont choisis dans le groupe constitué par l'hydrogène, -(CH₂)ₙ-COOH, n étant un entier de 1 à 6, et p un entier valant 2 ou 3 ;
R est un acide aminé tel que Thr, D- ou L-Phe ou bien D- ou L-Phe substitué en ortho et/ou en para par des groupes -OR', R' étant un groupe alkyle linéaire ou ramifié, benzyle ou benzyle substitué sur le cycle aromatique par des groupes hydrophobes.

2. Composé de formule (I) selon la revendication 1, dans lequel le symbole ------ est une liaison simple.

3. Composé de formule (I) selon la revendication 1, dans lequel le symbole ------ est une double liaison.

4. Composé selon les revendications 1 à 3, dans lequel le macrocycle de formule (II) est l'agent chélatant DOTA.

5. Procédé de préparation de composés selon les revendications 1 à 4, dans lequel -A est H, dans lequel :
(a) dans la première étape, le peptide linéaire est préparé par synthèse de peptide en phase solide tel que connu dans le domaine technique ;
(b) après la déprotection, le peptide linéaire sur la résine est cyclisé par un catalyseur de Grubbs de première génération ;
(c) le cyclopeptide est clivé de la résine et purifié par RP-HPLC ;
éventuellement
(d) après le clivage, le cyclopeptide est réduit en solution par H₂ en présence d'un catalyseur métallique et purifié par RP-HPLC ;
ou après l'étape (b)
(e) le cyclopeptide est réduit sur la résine par H₂ en présence d'un catalyseur métallique
(f) le cyclopeptide réduit est clivé de la résine et purifié par RP-HPLC.

6. Procédé de préparation de composés selon les revendications 1 à 4, dans lequel -A est un macrocycle de formule (II), dans lequel :
(a) dans la première étape, le peptide linéaire est préparé par synthèse de peptide en phase solide ;
(b) après la déprotection, le peptide linéaire sur la résine est cyclisé par un catalyseur de Grubbs de première génération ;
(c) le cyclopeptide insaturé est traité par la pipéridine ;
(d) le cyclopeptide est conjugué avec une solution du macrocycle activé de formule (II) ;
(e) le composé bioconjugué est clivé de la résine et purifié par RP-HPLC ;
ou, après l'étape (b) :
(c') - le cyclopeptide est réduit sur la résine ;
(d') - le cyclopeptide saturé est traité par la pipéridine ;
(e') - le cyclopeptide est conjugué avec le macrocycle ;
(f) le composé bioconjugué est clivé de la résine et purifié par RP-HPLC.

7. Formulations pharmaceutiques contenant en tant que principe actif un composé de formule (I).

8. Utilisation d'un composé de formule (I) pour la préparation de formulations pharmaceutiques utiles dans le traitement de tumeurs, de l'acromégalie, du diabète, de la polyarthrite rhumatoïde et de la maladie d'Alzheimer.
